# EUROPÄISCHE PATENTSCHRIFT

(11) **EP 3 189 365 B1**
(45) Veröffentlichungstag und Bekanntmachung des Hinweises auf die Patenterteilung: **25.01.2023**
(21) Anmeldenummer: 15762929.6
(22) Anmeldetag: 20.08.2015
(51) Int. Cl.: A61B 90/70, G02B 27/01, A61B 90/96, A61B 90/98

(54) **BENUTZERASSISTENZSYSTEM EINER REINIGUNGS- UND DESINFEKTIONSEINRICHTUNG**
USER ASSISTANCE SYSTEM COMPRISING A CLEANING AND DISINFECTING DEVICE
SYSTÈME D'ASSISTANCE À UN UTILISATEUR D'UN DISPOSITIF DE NETTOYAGE ET DE DÉSINFECTION

(30) Priorität: 03.09.2014 DE 102014217559
(43) Veröffentlichungstag der Anmeldung: 12.07.2017
(73) Patentinhaber: Olympus Winter & Ibe GmbH, 22045 Hamburg (DE)
(72) Erfinder: CARLSON, Torben, 22047 Hamburg (DE)
(74) Vertreter: Seemann & Partner Patentanwälte mbB
(86) Internationale Anmeldenummer: PCT/EP2015/069123
(87) Internationale Veröffentlichungsnummer: WO 2016/034422

(56) Entgegenhaltungen:
- JP-A- 2009 291 308
- JP-A- 2013 106 790
- US-A1- 2002 161 460
- US-A1- 2013 278 631

## Beschreibung

### Stand der Technik

Die Patentdokumente JP 2009 291308 A, US 2002/161460 A1, JP 2013 106790 A und US 2013/278631 A1 offenbaren relevanten Stand der Technik.

### Beschreibung

Die Erfindung betrifft ein Benutzerassistenzsystem einer Reinigungs- und Desinfektionseinrichtung zum Reinigen und Desinfizieren von chirurgischen Instrumenten nach Anspruch 1. Ferner betrifft die Erfindung ein Verfahren zum Betreiben eines solchen Benutzerassistenzsystems nach Anspruch 10.

Die Erfindung betrifft ebenso eine Reinigungs- und Desinfektionseinrichtung umfassend ein Benutzerassistenzsystem sowie ein Computerprogrammprodukt nach Anspruch 13.

An die Aufbereitung, d.h. die Reinigung und Desinfektion, von chirurgischen Instrumenten werden hohe Anforderungen gestellt. Eine erfolgreiche Aufbereitung setzt voraus, dass das chirurgische Instrument, beispielweise ein Endoskop, entsprechend den Herstellervorgaben in die Reinigungs- und Desinfektionseinrichtung eingelegt und falls erforderlich an deren Anschlüsse angeschlossen wird. Die Vielfalt von im klinischen Umfeld verwendeten chirurgischen Instrumenten erfordert ein großes Know-how bei den mit der Aufbereitung beauftragten Personen. Beispielsweise müssen Endoskope unterschiedlicher Typen verschieden in den Reinigungskorb der Reinigungs- und Desinfektionseinrichtung eingelegt und an unterschiedliche Anschlüsse angeschlossen werden. Zur Unterstützung der mit der Reinigung beauftragten Personen, werden die verschiedenen Anschlüsse und Einlegepositionen in einem Benutzerhandbuch der Reinigungs- und Desinfektionseinrichtung ausführlich beschrieben. Zusätzlich sind Kurzanleitungen in Form laminierter Handzettel weit verbreitet. Die gleichzeitige Handhabung eines zu reinigenden chirurgischen Instruments und eines Handzettels sind jedoch umständlich. Ferner ist weder die Aktualität noch die Verfügbarkeit einer solchen Kurzanleitung sichergestellt.

Es ist eine Aufgabe der Erfindung, ein Benutzerassistenzsystem einer Reinigungs- und Desinfektionseinrichtung, eine Reinigungs- und Desinfektionseinrichtung, ein Verfahren zum Betreiben eines Benutzerassistenzsystems sowie ein Computerprogrammprodukt anzugeben, wobei die Bedienung der Reinigungs- und Desinfektionseinrichtung vereinfacht sein soll.

Die Aufgabe wird gelöst durch ein Benutzerassistenzsystem einer Reinigungs- und Desinfektionseinrichtung zum Reinigen und Desinfizieren von chirurgischen Instrumenten, wobei das Benutzerassistenzsystem dadurch fortgebildet ist, dass das Benutzerassistenzsystem eine Datenbrille umfasst, die eine Kamera und ein bildgebendes optisches Modul mit einer Projektionsfläche umfasst, wobei das bildgebende optische Modul dazu eingerichtet ist, Informationen auf die im Blickfeld eines Benutzers der Datenbrille angeordnete Projektionsfläche zu projizieren und wobei das Benutzerassistenzsystem ferner einen Datenspeicher umfasst, auf welchem eine Vielzahl von Datensätzen für verschiedene Typen von chirurgischen Instrumenten gespeichert sind, wobei in jedem Datensatz Informationen betreffend ein Identifikationsmerkmal eines bestimmten Typs von chirurgischem Instrument und visuelle Daten betreffend Anweisungen zur Reinigung dieses Typs vorhanden sind, und wobei das Benutzerassistenzsystem ferner dazu eingerichtet ist, ein Bild eines zu reinigenden chirurgischen Instruments mit der Kamera zu erfassen, die Bilddaten des Bildes mit den Identifikationsmerkmalen in den Datensätze abzugleichen, einen Typ des zu reinigenden chirurgischen Instruments anhand eines in dem Bild vorhandenen Identifikationsmerkmals zu identifizieren und visuelle Informationen betreffend Anweisungen zur Reinigung dieses Typs mit dem optischen Modul auf der Projektionsfläche anzuzeigen.

Vorteilhaft hat die mit der Reinigung und Desinfektion beauftragte Person, beispielsweise ein Krankenhausmitarbeiter, bei der Vorbereitung des chirurgischen Instruments auf die Aufbereitung beide Hände frei, um das chirurgische Instrument korrekt und sicher einzulegen bzw. anzuschließen. Die Notwendigkeit, während dieser Tätigkeit gleichzeitig einen Handzettel oder ein Handbuch zu beachten, entfällt vorteilhaft. Der Krankenhausmitarbeiter hat das zu reinigende chirurgische Instrument und gleichzeitig Informationen betreffend dessen Handhabung im Blickfeld.

Bei den zur Verfügung gestellten Informationen handelt es sich beispielsweise um einen erklärenden Text und/oder um ein Bild oder eine Skizze, welcher/welche die durchzuführenden Handgriffe erläutern. Auch eine Handlungsanweisung im Sinne einer erweiterten Realität (engl.: augmented reality) ist vorgesehen. So werden bevorzugt ein maschinenseitig zu verwendender Anschluss und ein instrumentenseitiger Anschlussstecker hervorgehoben. Beispielsweise werden die relevanten Bereiche farblich hervorgehoben oder mit Leuchtrahmen markiert. Es ist ebenso vorgesehen, dass die korrekte Position des chirurgischen Instruments dem für den Benutzer sichtbaren Bild überlagert wird. Ein virtuelles Instrument oder seine vereinfachte oder schematische Darstellung werden beispielsweise in das reale Bild eingeblendet bzw. diesem überlagert.

Das Benutzerassistenzsystem vereinfacht und beschleunigt die Aufbereitung chirurgischer Instrumente. Gleichzeitig wird der Benutzer in der Handhabung und Bedienung geführt und unterstützt, so dass der Aufbereitungsvorgang insgesamt weniger fehleranfällig ist.

Ein weiterer Vorteil ist, dass die Datensätze betreffend die Reinigung der verschiedenen Typen von chirurgischen Instrumenten auf einer elektronischen Datenbank hinterlegt sind. Eine Aktualisierung dieser Daten ist schnell, einfach und problemlos möglich, indem eine oder mehrere auf dem Datenspeicher gespeicherte Dateien aktualisiert werden. Dieses Verfahren ist dem Austausch einer Vielzahl von Handzetteln in Geschwindigkeit und Kosten deutlich überlegen.

Das erfindungsgemäße Benutzerassistenzsystem ist dadurch fortgebildet, dass es die Merkmale des Anspruchs 1 umfasst.

Verschiedene Typen von Aufnahmevorrichtungen, wie sie beispielsweise in verschiedenen Baureihen oder Typen von Reinigungs- und Desinfektionseinrichtungen zum Einsatz kommen, erfordern eine unterschiedliche Handhabung der zu reinigenden chirurgischen Instrumente. Beispielsweise sind die Anschlüsse, an die das chirurgische Instrument zur Aufbereitung anzuschließen ist, an unterschiedlichen Positionen vorhanden. Ferner wird das chirurgische Instrument vielfach in unterschiedliche Typen von Aufnahmevorrichtungen unterschiedlich eingelegt.

Vorteilhaft wird bei einem Benutzerassistenzsystem gemäß der genannten Ausführungsform nicht nur der Typ des chirurgischen Instruments, sondern auch der Typ der Aufnahmevorrichtung erkannt. Es werden Informationen betreffend die Handhabung des erkannten Typs des chirurgischen Instruments bei Verwendung des erkannten Typs der Aufnahmevorrichtung zur Verfügung gestellt. Dem Krankenhausmitarbeiter liegen alle notwendigen Informationen vor, um das zu reinigende chirurgische Instrument korrekt in der Aufnahmevorrichtung zu platzieren. Eine effektive Reinigung und Desinfektion ist sichergestellt.

Gemäß einer Ausführungsform ist das Benutzerassistenzsystem dadurch fortgebildet, dass das Identifikationsmerkmal zumindest ein Teil einer äußeren Erscheinung und/oder einer Form des chirurgischen Instruments oder der Aufnahmevorrichtung, und/oder das Identifikationsmerkmal zumindest ein Teil eines an oder auf dem chirurgischen Instrument oder der Aufnahmevorrichtung vorhandenen maschinenlesbaren Codes ist.

Bei dem maschinenlesbaren Code handelt es sich insbesondere um einen Barcode und/oder einen QR-Code. Vorteilhaft ist die Datenbrille derart eingerichtet, dass sie ausgehend von den erfassten Bilddaten anhand eines oder mehrerer der genannten Identifikationsmerkmale den Typ des chirurgischen Instruments und/oder der Aufnahmevorrichtung selbstständig identifiziert. Eine weitere Handlung des Benutzers ist nicht notwendig. Dieser wird in die Lage versetzt, sich auf seine eigentlichen Aufgaben, nämlich die korrekte Handhabung des chirurgischen Instruments, zu konzentrieren.

Das erfindungsgemäße Benutzerassistenzsystem ist anderen elektronischen Benutzerassistenzsystemen hinsichtlich seiner Benutzerfreundlichkeit überlegen. Vorteilhaft entfällt die Notwendigkeit, eine Eingabeeinheit, beispielsweise ein Touchpad oder eine Tastatur, zu bedienen. Auch die Verwendung eines Scanners, beispielsweise zur Erfassung eines Barcodes, entfällt vorteilhaft. Bei den genannten Lösungen besteht stets die Gefahr, dass diese Einheiten in Kontakt mit dem verunreinigten chirurgischen Instrument gelangen, was unerwünscht ist. Durch den Einsatz einer Datenbrille in dem Benutzerassistenzsystem wird dies vorteilhaft vermieden.

Gemäß einer Weiterbildung des Benutzerassistenzsystems ist vorgesehen, dass die visuellen Informationen das Einlegen des chirurgischen Instruments in die Aufnahmevorrichtung und/oder das Anschließen des chirurgischen Instruments an Anschlüsse der Aufnahmevorrichtung betreffen.

Diese Ausführungsform ist insbesondere für Endoskope als chirurgische Instrumente vorteilhaft. Sie erleichtert der mit der Reinigung und Desinfektion beauftragten Person die Aufbereitung des Endoskops. Endoskope verfügen über eine Vielzahl von Anschlüssen, welche vor der Reinigung und Desinfektion an verschiedene Schläuche oder Anschlussstutzen der Reinigungs- und Desinfektionseinrichtung angeschlossen werden müssen. Aufgrund der Vielfalt der im klinischen Umfeld vorhandenen Endoskope, stellt die korrekte Zuordnung der Anschlüsse das Klinikpersonal regelmäßig vor Probleme. Bei dem Benutzerassistenzsystem gemäß der genannten Ausführungsform werden dem Krankenhausmitarbeiter alle notwendigen Informationen zur Verfügung gestellt, so dass ein korrektes Anschließen des Endoskops vereinfacht ist.

Gemäß der beanspruchten Erfindung ist das Benutzerassistenzsystem dadurch fortgebildet, dass die Datenbrille ferner eine Audioausgabevorrichtung umfasst und in zumindest einem Datensatz und/oder zumindest einem weiteren Datensatz die visuellen Daten ergänzende Audiodaten umfassend Anweisungen zur Reinigung des chirurgischen Instruments vorhanden sind.

Die genannten Audiodateien umfassen Anweisungen zur Handhabung und/oder Reinigung des chirurgischen Instruments und unterstützen die visuelle Darstellung. Insbesondere werden die Audioanweisungen synchronisiert mit den visuellen Darstellungen betreffend die Handhabung des chirurgischen Instruments ausgegeben. So ergänzen sich vorteilhaft die zur Verfügung gestellten visuellen Daten und beispielsweise gesprochene Handlungsanweisung. Die Handhabung des chirurgischen Instruments im Hinblick auf seine Aufbereitung wird nochmals vereinfacht.

In einer weiteren Ausführungsform ist ferner vorgesehen, dass die Projektionsfläche ein Teil eines, insbesondere transparenten, Prismas ist, wobei für einen Benutzer der Datenbrille in Blickrichtung durch den die Projektionsfläche umfassenden Teil des Prismas sowohl das Blickfeld als auch auf die Projektionsfläche projizierte Informationen beobachtbar sind.

Als Material für das Prisma ist insbesondere Glas oder ein transparenter Kunststoff, beispielsweise Plexiglas, vorgesehen. Vorteilhaft verdeckt ein solches Prisma das Blickfeld des Benutzers nicht. Dieser blickt ungehindert durch das Prisma hindurch und behält die im eigenen Blickfeld vorhandenen Gegenstände ungestört im Auge, während er mit den zur Aufbereitung des chirurgischen Instruments notwendigen Informationen versorgt wird.

Gemäß einer weiteren Ausführungsform ist vorgesehen, dass der Datenspeicher von der Datenbrille umfasst ist. Bei dem Datenspeicher handelt es sich insbesondere um einen nichtflüchtigen Datenspeicher. Gemäß einer alternativen Ausführungsform ist vorgesehen, dass der Datenspeicher außerhalb der Datenbrille vorhanden ist und die Datenbrille eine Sende-/Empfangseinheit zur Herstellung einer drahtgebundenen oder drahtlosen Datenverbindung mit dem Datenspeicher umfasst.

Insbesondere ist der Datenspeicher in einer zentralen Datenverarbeitungseinheit (Server) vorhanden. Bei dem Datenspeicher handelt es sich insbesondere um einen zentralen Datenspeicher, auf den vorteilhaft eine Vielzahl von Datenbrillen gegebenenfalls verschiedener Benutzerassistenzsysteme zugreift. Die Datenbrillen sind ferner insbesondere unterschiedlichen Reinigungs- und Desinfektionseinrichtungen zugeordnet. Eine zentrale Verwaltung der Informationen betreffend die Handhabung verschiedener chirurgischer Instrumente und/oder verschiedener Aufnahmevorrichtungen bietet den Vorteil, dass eine Aktualisierung der Daten mit sehr geringem Aufwand vorgenommen werden kann.

Insbesondere ist das Benutzerassistenzsystem dadurch fortgebildet, dass das chirurgische Instrument ein Endoskop ist. Endoskope als chirurgische Instrumente sind in einer Vielzahl verschiedener Typen für verschiedene Anwendungen verfügbar und erfordern eine teils erheblich unterschiedliche Handhabung. Zur korrekten Aufbereitung von Endoskopen sind eine Vielzahl einzelner Schritte durchzuführen und verschiedenste Details bezüglich ihrer Handhabung zu beachten. Dies erfordert, dass zu jedem Endoskop ein Benutzerhandhandbuch vorliegt und Beachtung findet. Indem die für die Aufbereitung relevanten Informationen in einer Datenbank zusammengefasst und dem Benutzer in komfortabler Weise zur Verfügung gestellt werden, ist die Aufbereitung von Endoskopen wesentlich vereinfacht und außerdem zuverlässiger und sicherer.

Die Datenbrille des Benutzerassistenzsystems bietet ihrem Benutzer die Funktionalität eines Head-up-Displays. Es handelt sich bei der Datenbrille wahlweise um eine monokulare oder eine binokulare Datenbrille. Mit anderen Worten werden die zur Verfügung gestellten Informationen also in das Blickfeld eines oder beider Augen des Benutzers eingeblendet. Die Datenbrille ist ihrer Funktionalität nach ein am Kopf tragbarer Kleincomputer. Sie umfasst insbesondere einen Zentralprozessor (CPU), einen Arbeitsspeicher (RAM), ein Mikrofon, eine in Blickrichtung gerichtete Digitalkamera, einen Lautsprecher, beispielsweise einen Knochenleitungs-Lautsprecher, eine Antenne zum Empfang von Signalen über eine drahtlose Datenübertragungsstrecke, beispielsweise eine Antenne zum Empfang von Signalen über Bluetooth, WLAN, UMTS, 3G, etc., einen Beschleunigungssensor und einen Energiespeicher (Akku).

Der Zentralprozessor, beispielsweise ein Mikroprozessor, ist dazu eingerichtet, die erfassten Bilddaten zu verarbeiten und das bildgebende optische Modul anzusteuern. Befindet sich der Datenspeicher in der Datenbrille, so ist der Zentralprozessor dazu eingerichtet, in den erfassten Bilddaten zumindest ein Identifikationsmerkmal zu identifizieren, aus dem Datenspeicher Datensätzen abzurufen und die dort vorhandenen Identifikationsmerkmale abzugleichen. Anschließend ruft der Zentralprozessor die visuellen Daten und/oder die Audiodaten aus dem zugehörigen Datensatz ab, und stellt diese dem bildgebenden optischen Modul und/oder der Audioausgabeeinheit zur Verfügung.

Befindet sich der Datenspeicher in einem zentralen Server, so ist der Zentralprozessor der Datenbrille dazu eingerichtet, wahlweise die erfassten Bilddaten an den Server zu übertragen oder Datensätze von diesem anzufordern und zu empfangen. Eine Analyse der erfassten Bilddaten findet wahlweise im Zentralprozessor der Datenbrille oder auf dem Server statt. Abhängig davon, ob die Analyse dezentral oder zentral durchgeführt wird, erfolgt auch ein Abgleich des Identifikationsmerkmals mit den gespeicherten Identifikationsmerkmalen dezentral oder zentral. Anschließend werden die visuellen Daten und/oder die Audiodaten aus dem zugehörigen Datensatz abgerufen, an die Datenbrille übertragen und dem bildgebenden optischen Modul und/oder der Audiowiedergabeeinheit zur Verfügung gestellt.

Die oben genannten Aspekte betreffen vorteilhaft alle Ausführungsformen.

Die erfindungsgemäße Aufgabe wird ferner gelöst durch eine Reinigungs- und Desinfektionseinrichtung, umfassend ein Benutzerassistenzsystem nach einer oder mehreren der genannten Ausführungsformen. Für die Reinigungs- und Desinfektionseinrichtung treffen gleiche oder ähnliche Vorteile zu, wie sie bereits im Hinblick auf das Benutzerassistenzsystem erwähnt wurden.

Die erfindungsgemäße Aufgabe wird ferner gelöst durch ein Verfahren zum Betreiben eines Benutzerassistenzsystems nach einem oder mehreren der genannten Ausführungsformen, wobei das Verfahren die in Anspruch 10 offenbarten Schritte umfasst.

Auch auf das Verfahren zum Betreiben eines Benutzerassistenzsystems treffen gleiche oder ähnliche Vorteile zu, wie sie bereits im Hinblick auf das Benutzerassistenzsystem erwähnt wurden.

Gemäß einer Ausführungsform ist vorgesehen, dass das Verfahren dadurch fortgebildet ist, dass auf dem Datenspeicher ferner eine Vielzahl von weiteren Datensätzen für verschiedene Typen von Aufnahmevorrichtungen, insbesondere Reinigungskörben von Reinigungs- und Desinfektionseinrichtungen, gespeichert sind, wobei in jedem dieser Datensätze Informationen betreffend ein Identifikationsmerkmal eines bestimmten Typs von Aufnahmevorrichtung und visuelle Informationen betreffend die Reinigung chirurgischer Instrumente unter Verwendung dieses Typs vorhanden sind, wobei das Verfahren ferner die folgenden Schritte umfasst:
- Erfassen eines zusätzlichen Bildes einer Aufnahmevorrichtung mit der Kamera,
- Abgleichen der Bilddaten des zusätzlichen Bildes mit den Identifikationsmerkmalen in den weiteren Datensätzen,
- Identifizieren eines Typs der Aufnahmevorrichtung anhand eines in dem zusätzlichen Bild vorhandenen Identifikationsmerkmals und
- Anzeigen visueller Informationen betreffend die Reinigung des chirurgischen Instruments unter Verwendung dieses Typs von Aufnahmevorrichtung mit dem optischen Modul auf der Projektionsfläche.

Gemäß einer weiteren Ausführungsform ist das Verfahren zum Betreiben eines Benutzerassistenzsystems dadurch fortgebildet, dass das Identifikationsmerkmal zumindest ein Teil einer äußeren Erscheinung und/oder einer Form des chirurgischen Instruments und/oder zumindest ein Teil eines an oder auf dem chirurgischen Instrument vorhandenen maschinenlesbaren Codes ist.

Das erfindungsgemäße Verfahren zum Betreiben eines Benutzerassistenzsystems ist dadurch fortgebildet, dass die Datenbrille ferner eine Audioausgabevorrichtung umfasst und in zumindest einem Datensatz und/oder zumindest einem weiteren Datensatz die visuellen Daten ergänzende Audiodaten umfassend Anweisungen zur Reinigung des chirurgischen Instruments vorhanden sind und visuellen Informationen ergänzende Audiodaten ausgegeben werden.

Auch für die genannten vorteilhaften Ausführungsformen des Verfahrens treffen gleiche oder ähnliche Vorteile zu, wie sie bereits im Hinblick auf die analogen Ausführungsformen des Benutzerassistenzsystems erwähnt wurden.

Die erfindungsgemäße Aufgabe wird außerdem gelöst durch ein Computerprogrammprodukt, welches eine Datenbrille in einem Benutzerassistenzsystem nach einem oder mehreren der genannten Ausführungsformen dazu veranlasst, ein Verfahren nach einer oder mehreren der genannten Ausführungsformen auszuführen.

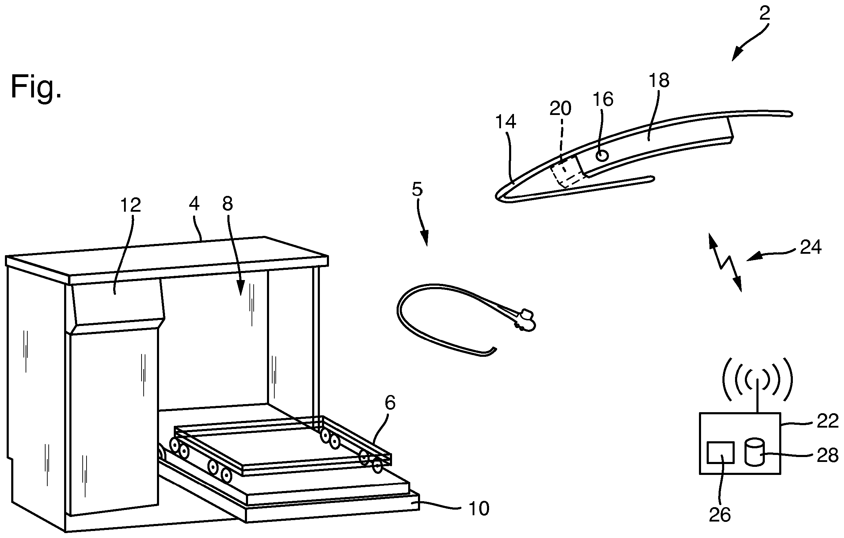

Weitere Merkmale der Erfindung werden aus der Beschreibung erfindungsgemäßer Ausführungsformen zusammen mit den Ansprüchen und der beigefügten Zeichnung ersichtlich.

Die Erfindung wird nachstehend ohne Beschränkung des allgemeinen Erfindungsgedankens anhand von Ausführungsbeispielen unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt deren einzige Figur ein schematisches Benutzerassistenzsystem einer Reinigungs- und Desinfektionseinrichtung mit einer Datenbrille.

In der Zeichnung sind jeweils gleiche oder gleichartige Elemente und/oder Teile mit denselben Bezugsziffern versehen, so dass von einer erneuten Vorstellung jeweils abgesehen wird.

Die Figur zeigt ein Benutzerassistenzsystem 2 einer Reinigungs- und Desinfektionseinrichtung 4. Die Reinigungs- und Desinfektionseinrichtung 4 dient der Aufbereitung, d.h. der Reinigung und Desinfektion, von chirurgischen Instrumenten 5. Insbesondere ist die Reinigungs- und Desinfektionseinrichtung 4 zur Aufbereitung von Endoskopen eingerichtet.

Bevor die chirurgischen Instrumente 5 aufbereitet werden, werden diese in eine Aufnahmevorrichtung 6, bei der es sich beispielhaft um einen Reinigungskorb handelt, eingelegt. Ferner werden die chirurgischen Instrumente 5, vor allem wenn es sich bei diesen um Endoskope handelt, an in der Figur nicht dargestellte Anschlüsse der Reinigungs- und Desinfektionseinrichtung 4 angeschlossen. Anschließend erfolgt die Aufbereitung der chirurgischen Instrumente 5 in einem Spülraum 8, der mit der Tür 10 verschlossen wird. Die Parameter zur Reinigung und Desinfektion der chirurgischen Instrumente 5 werden, sofern notwendig, über ein Bedienfeld 12 an der Vorderseite der Reinigungs- und Desinfektionseinrichtung 4 eingegeben.

Das Benutzerassistenzsystem 2 umfasst eine Datenbrille 14. Beispielhaft ist eine monokulare Datenbrille 14 dargestellt. Ebenso ist eine binokulare Datenbrille 14 verwendbar. Die Datenbrille 14 umfasst eine Kamera 16, deren Aufnahmerichtung in Blickrichtung eines Benutzers der Datenbrille 14 gerichtet ist. Ferner umfasst die Datenbrille 14 ein bildgebendes optisches Modul, welches innerhalb eines Gehäuses 18 angeordnet ist. Das bildgebende optische Modul umfasst eine bilderzeugende Einheit, beispielsweise einen Mikroprojektor, optische Einheiten, wie beispielsweise einen Kollimator und ein Prisma 20, welches eine Projektionsfläche umfasst. Beispielsweise handelt es sich bei der Projektionsfläche um eine Außenseite des Prismas 20. Das Prisma 20 ist beispielsweise aus Glas oder Plexiglas hergestellt, so dass ein Benutzer der Datenbrille ungehindert durch das Prisma 20 hindurchblicken kann.

Eine mit der Reinigung des chirurgischen Instruments 5 befasste Person bedient sich des Benutzerassistenzsystems 2, indem sie zunächst die Datenbrille 14 aufsetzt und das chirurgische Instrument 5 betrachtet. Es wird ein Bild des chirurgischen Instruments 5 mithilfe der Kamera 16 erfasst. Die Bilddaten dieses Bildes werden im Hinblick auf ein Identifikationsmerkmal des chirurgischen Instruments 5 analysiert. Das Identifikationsmerkmal ist insbesondere zumindest ein Teil einer äußeren Erscheinung und/oder eine Form des chirurgischen Instruments 5. Ferner handelt es sich bei dem Identifikationsmerkmal insbesondere um zumindest einen Teil eines maschinenlesbaren Codes, beispielweise eines Barcodes oder eines QR-Codes.

In dem in der Figur dargestellten Ausführungsbeispiel findet die Bildanalyse in einem Server 22 statt, der über eine drahtlose Datenverbindung 24 mit der Datenbrille 14 in Kontakt steht. Zu diesem Zweck ist die Datenbrille 14 mit einer Sende-/Empfangseinheit ausgestattet. Gleiches gilt für den Server 22. Die mit der Kamera 16 erfassten Bilddaten werden über die drahtlose Datenverbindung 24 in den Server 22 übertragen und dort in einem Zentralprozessor 26 verarbeitet.

Der Server 22 umfasst ferner einen Datenspeicher 28, auf welchem eine Vielzahl von Datensätzen für verschiedene Typen von chirurgischen Instrumenten 5 gespeichert sind. Jeder dieser Datensätze umfasst Informationen betreffend ein Identifikationsmerkmal eines bestimmten Typs von chirurgischem Instrument 5. Beispielsweise sind in einem solchen Datensatz Informationen betreffend eine äu-ßere Erscheinung und/oder eine Form verschiedener Typen von chirurgischen Instrumenten 5 vorhanden. Es ist ferner möglich, dass chirurgische Instrumente 5 identischen Typs mit einem gleichen maschinenlesbaren Code versehen werden, so dass sie auf diesem Wege als chirurgische Instrumente 5 des gleichen Typs identifizierbar sind.

In den auf dem Datenspeicher 28 vorhandenen Datensätzen sind ferner Anweisungen zur Reinigung chirurgischer Instrumente 5 unterschiedlicher Typen vorhanden. Insbesondere handelt es sich bei diesen Informationen um visuelle Daten. Geeignet sind Bilder oder Skizzen oder auch bewegte Animationen oder Filmsequenzen, welche die Bedienung des identifizierten Typs zur Vorbereitung der Reinigung und Desinfektion zeigen. Es wird beispielsweise erklärt, wie der identifizierte Typ Endoskop in die Aufnahmevorrichtung 6 der Reinigungs- und Desinfektionseinrichtung 4 einzulegen ist und wie welche Anschlüsse des Endoskops mit welchen Anschlüssen der Reinigungs- und Desinfektionseinrichtung 4 zu verbinden sind.

Die Bilddaten werden über die drahtlose Datenverbindung 24 von dem Server 22 zu der Datenbrille 14 übertragen. Diese stellt die Bilddaten mit Hilfe des optischen Moduls auf der Projektionsfläche des Prismas 20 dar.

Der Benutzer des Benutzerassistenzsystems 2, welcher die Datenbrille 14 trägt, hat sowohl das chirurgische Instrument 5, die Aufnahmevorrichtung 6 als auch die von der Datenbrille 14 zusätzlich zur Verfügung gestellten Informationen im Blickfeld. Gleichzeitig behält er beide Hände frei, so dass das Einlegen und Anschließen des chirurgischen Instruments 5 an die Reinigungs- und Desinfektionsreinrichtung 4 schnell und sicher durchführbar ist.

Auf dem Datenspeicher 28 ist gemäß einem weiteren Ausführungsbeispiel eine Vielzahl weiterer Datensätze vorhanden. Diese weiteren Datensätze betreffen verschiedene Typen von Aufnahmevorrichtungen 6. Beispielsweise umfassen die Datensätze Informationen betreffend die Handhabung verschiedener Reinigungskörbe verschiedener Reinigungs- und Desinfektionseinrichtungen 4. Mit Hilfe der Datenbrille 14, genauer deren Kamera 16, wird ein Identifikationsmerkmal der Aufnahmevorrichtung 6 erfasst. Ähnlich wie bei dem chirurgischen Instrument 5 handelt es sich bei diesem Identifikationsmerkmal beispielsweise um zumindest einen Teil der äußeren Erscheinung und/oder einer Form der Aufnahmevorrichtung 6. Ferner ist insbesondere vorgesehen, dass die Aufnahmevorrichtung 6 mit einem maschinenlesbaren Code versehen ist, welcher zumindest teilweise als Identifikationsmerkmal dient.

Bilddaten eines Bildes der Aufnahmevorrichtung 6 werden von der Datenbrille 14 über die drahtlose Datenverbindung 24 an den Server 22 übertragen. Dort führt der Zentralprozessor 26 einen Abgleich mit in den weiteren Datensätzen vorhandenen Identifikationsmerkmalen durch und bestimmt einen Typ der Aufnahmevorrichtung 6. Anschließend werden visuelle Informationen betreffend die Reinigung des chirurgischen Instruments 5 unter Verwendung dieses speziellen Typs von Aufnahmevorrichtung 6 über die drahtlose Datenverbindung 24 an die Datenbrille 14 übertragen. Diese stellt unter Verwendung ihres optischen Moduls diese Informationen auf der Projektionsfläche dar.

Insbesondere ist vorgesehen, dass die Informationen betreffend die Handhabung der Aufnahmevorrichtung 6 mit den Informationen betreffend die Handhabung des chirurgischen Instruments 5 kombiniert werden. Der Benutzer erhält mit anderen Worten spezifische Anweisungen, wie der erkannte Typ eines chirurgischen Instruments 5 unter Verwendung der erkannten Aufnahmevorrichtung 6 zu bedienen ist. Beispielsweise wird dem Benutzer angezeigt, wie ein spezieller Typ Endoskop in einen bestimmten Reinigungskorb einer Reinigungs- und Desinfektionseinrichtung 4 eingelegt wird und welche Anschlüsse des Endoskops mit welchen Verbindungsschläuchen oder Stutzen der Reinigungs- und Desinfektionseinrichtung 4 zu verbinden sind.

Die visuellen Daten werden bevorzugt durch Audiodaten, beispielsweise gesprochene Anweisungen, unterstützt. Zu diesem Zweck liegen auf dem Datenspeicher 28 neben den visuellen Daten diese ergänzende Audiodaten vor. Die ergänzenden Audiodaten werden gemeinsam mit den Daten betreffend die visuellen Anweisungen über die drahtlose Datenverbindung 24 übertragen. Beispielsweise werden dem Benutzer gemeinsam mit erläuternden Skizzen Audiokommentare oder Anweisungen zur Verfügung gestellt. Zur Wiedergabe derselben verfügt die Datenbrille 14 über eine Audioausgabevorrichtung, beispielsweise einen Lautsprecher oder einen Knochenleitungs-Lautsprecher.

Gemäß einem weiteren Ausführungsbeispiel befindet sich der Datenspeicher 28 und der Zentralprozessor 26 innerhalb des Gehäuses 18 der Datenbrille 14. Die Auswertung der erfassten Bilddaten und der Abgleich mit auf dem Datenspeicher 28 vorhandenen Datensätzen erfolgt innerhalb der Datenbrille 14.

Ferner ist insbesondere vorgesehen, dass der Server 22 mit mehreren Datenbrillen 14 in Verbindung steht. Vorteilhaft steht der Datenspeicher 28 somit einer Vielzahl von Datenbrillen 14 zur Verfügung, so dass durch Aktualisierung der Daten auf diesem zentralen Datenspeicher 28 dafür gesorgt ist, dass alle Datenbrillen auf aktuelle Datensätze zugreifen.

Gemäß einem alternativen Ausführungsbeispiel erfolgt eine Aktualisierung der auf dem Datenspeicher 28 vorhanden Daten, wenn dieser dezentral als Teil der Datenbrille 14 vorgesehen ist, über die drahtlose Datenverbindung 24, ausgehend vom Server 22.

Im Rahmen der Erfindung sind Merkmale, die mit "insbesondere" oder "vorzugsweise" gekennzeichnet sind, als fakultative Merkmale zu verstehen.

### Bezugszeichenliste

- 2: Benutzerassistenzsystem
- 4: Reinigungs- und Desinfektionseinrichtung
- 5: chirurgisches Instrument
- 6: Aufnahmevorrichtung
- 8: Spülraum
- 10: Tür
- 12: Bedienfeld
- 14: Datenbrille
- 16: Kamera
- 18: Gehäuse
- 20: Prisma
- 22: Server
- 24: drahtlose Datenverbindung
- 26: Zentralprozessor
- 28: Datenspeicher

## Patentansprüche

1. Benutzerassistenzsystem (2) einer Reinigungs- und Desinfektionseinrichtung (4) zum Reinigen und Desinfizieren von chirurgischen Instrumenten (5), wobei das Benutzerassistenzsystem (2) eine Datenbrille (14) umfasst, die eine Kamera (16) und ein bildgebendes optisches Modul mit einer Projektionsfläche umfasst, wobei das bildgebende optische Modul dazu eingerichtet ist, Informationen auf die im Blickfeld eines Benutzers der Datenbrille (14) angeordnete Projektionsfläche zu projizieren, und wobei das Benutzerassistenzsystem (2) ferner einen Datenspeicher (28) umfasst, auf welchem eine Vielzahl von Datensätzen für verschiedene Typen von chirurgischen Instrumenten (5) gespeichert sind, wobei in jedem Datensatz Informationen betreffend ein Identifikationsmerkmal eines bestimmten Typs von chirurgischem Instrument (5) und visuelle Daten betreffend Anweisungen zur Reinigung dieses Typs vorhanden sind, und wobei das Benutzerassistenzsystem (2) ferner dazu eingerichtet ist, ein Bild eines zu reinigenden chirurgischen Instruments (5) mit der Kamera (16) zu erfassen, die Bilddaten des Bildes mit den Identifikationsmerkmalen in den Datensätzen abzugleichen, einen Typ des zu reinigenden chirurgischen Instruments (5) anhand eines in dem Bild vorhandenen Identifikationsmerkmals zu identifizieren und visuelle Informationen betreffend Anweisungen zur Reinigung dieses Typs mit dem optischen Modul auf der Projektionsfläche anzuzeigen, wobei die visuellen Informationen sind:
ein erklärender Text
und/oder ein Bild oder eine Skizze, welcher/welche die durchzuführenden Handgriffe erläutern
und/oder eine Handlungsanweisung im Sinne einer erweiterten Realität, bei der insbesondere relevante Bereiche farblich hervorgehoben und/oder mit Leuchtrahmen markiert werden und/oder eine korrekte Position des chirurgischen Instruments (5) dem für den Benutzer sichtbaren Bild überlagert wird,
**dadurch gekennzeichnet, dass**
die Datenbrille (14) ferner eine Audioausgabevorrichtung umfasst und in zumindest einem Datensatz und/oder zumindest einem weiteren Datensatz die visuellen Daten ergänzende Audiodaten umfassend Anweisungen zur Reinigung des chirurgischen Instruments (5) vorhanden sind.

2. Benutzerassistenzsystem (2) nach Anspruch 1, **dadurch gekennzeichnet, dass** auf dem Datenspeicher (28) ferner eine Vielzahl von weiteren Datensätzen für verschiedene Typen von Aufnahmevorrichtungen (6), insbesondere Reinigungskörben von Reinigungs- und Desinfektionseinrichtungen (4), gespeichert sind, wobei in jedem dieser Datensätze Informationen betreffend ein Identifikationsmerkmal eines bestimmten Typs von Aufnahmevorrichtung (6) und visuelle Informationen betreffend die Reinigung chirurgischer Instrumente (5) unter Verwendung dieses Typs vorhanden sind, wobei das Benutzerassistenzsystem (2) ferner dazu eingerichtet ist, zusätzlich ein Bild einer Aufnahmevorrichtung (6) mit der Kamera (16) zu erfassen, die Bilddaten des zusätzlichen Bildes mit den Identifikationsmerkmalen in den weiteren Datensätzen abzugleichen, einen Typ der Aufnahmevorrichtung (6) anhand eines in dem Bild vorhandenen Identifikationsmerkmals zu identifizieren und visuelle Informationen betreffend die Reinigung des chirurgischen Instruments (5) unter Verwendung dieses Typs von Aufnahmevorrichtung (6) mit dem optischen Modul auf der Projektionsfläche anzuzeigen.

3. Benutzerassistenzsystem (2) nach Anspruch 1 oder 2, **dadurch gekennzeichnet, dass** das Identifikationsmerkmal zumindest ein Teil einer äußeren Erscheinung und/oder einer Form des chirurgischen Instruments (5) oder der Aufnahmevorrichtung (6), und/oder das Identifikationsmerkmal zumindest ein Teil eines an oder auf dem chirurgischen Instrument (5) oder der Aufnahmevorrichtung (6) vorhandenen maschinenlesbaren Codes ist.

4. Benutzerassistenzsystem (2) nach Anspruch 2 oder 3, **dadurch gekennzeichnet, dass** die visuellen Informationen das Einlegen des chirurgischen Instruments (5) in die Aufnahmevorrichtung (6) und/oder das Anschließen des chirurgischen Instruments (5) an Anschlüsse der Aufnahmevorrichtung (6) betreffen.

5. Benutzerassistenzsystem (2) nach einem der Ansprüche 1 bis 4, **dadurch gekennzeichnet, dass** die Projektionsfläche ein Teil eines Prismas (20) ist, wobei für einen Benutzer der Datenbrille (14) in Blickrichtung durch den die Projektionsfläche umfassenden Teil des Prismas (20) sowohl das Blickfeld als auch auf die Projektionsfläche projizierte Informationen beobachtbar sind.

6. Benutzerassistenzsystem (2) nach einem der Ansprüche 1 bis 5, **dadurch gekennzeichnet, dass** der Datenspeicher (28) von der Datenbrille (14) umfasst ist.

7. Benutzerassistenzsystem (2) nach einem der Ansprüche 1 bis 5, **dadurch gekennzeichnet, dass** der Datenspeicher (28) außerhalb der Datenbrille (14) vorhanden ist und die Datenbrille (14) eine Sende-/Empfangseinheit zur Herstellung einer drahtgebundenen oder drahtlosen Datenverbindung (24) mit dem Datenspeicher (28) umfasst.

8. Benutzerassistenzsystem (2) nach einem der Ansprüche 1 bis 7, **dadurch gekennzeichnet, dass** das chirurgische Instrument (5) ein Endoskop ist.

9. Reinigungs- und Desinfektionseinrichtung (4), umfassend ein Benutzerassistenzsystem (2) nach einem der Ansprüche 1 bis 8.

10. Verfahren zum Betreiben eines Benutzerassistenzsystems (2) nach einem der Ansprüche 1 bis 8, wobei das Verfahren die folgenden Schritte umfasst:
- Erfassen eines Bildes eines zu reinigenden chirurgischen Instruments (5) mit der Kamera (16),
- Abgleichen der Bilddaten des Bildes mit den Identifikationsmerkmalen in den Datensätzen,
- Identifizieren eines Typs des zu reinigenden chirurgischen Instruments (5) anhand eines in dem Bild vorhandenen Identifikationsmerkmals,
- Anzeigen visueller Informationen betreffend Anweisungen zur Reinigung dieses Typs mit dem optischen Modul auf der Projektionsfläche,
**dadurch gekennzeichnet, dass**
die Datenbrille (14) ferner eine Audioausgabevorrichtung umfasst und in zumindest einem Datensatz und/oder zumindest einem weiteren Datensatz die visuellen Daten ergänzende Audiodaten umfassend Anweisungen zur Reinigung des chirurgischen Instruments (5) vorhanden sind und die visuellen Informationen ergänzende Audiodaten ausgegeben werden.

11. Verfahren zum Betreiben eines Benutzerassistenzsystems (2) nach Anspruch 10, **dadurch gekennzeichnet, dass** auf dem Datenspeicher (28) ferner eine Vielzahl von weiteren Datensätzen für verschiedene Typen von Aufnahmevorrichtungen (6), insbesondere Reinigungskörben von Reinigungs- und Desinfektionseinrichtungen (4), gespeichert sind, wobei in jedem dieser Datensätze Informationen betreffend ein Identifikationsmerkmal eines bestimmten Typs von Aufnahmevorrichtung (6) und visuelle Informationen betreffend die Reinigung chirurgischer Instrumente (5) unter Verwendung dieses Typs vorhanden sind, wobei das Verfahren ferner die folgenden Schritte umfasst:
- Erfassen eines zusätzlichen Bildes einer Aufnahmevorrichtung (6) mit der Kamera (16),
- Abgleichen der Bilddaten des zusätzlichen Bildes mit den Identifikationsmerkmalen in den weiteren Datensätzen,
- Identifizieren eines Typs der Aufnahmevorrichtung (6) anhand eines in dem Bild vorhandenen Identifikationsmerkmals und
- Anzeigen visueller Informationen betreffend die Reinigung des chirurgischen Instruments (5) unter Verwendung dieses Typs von Aufnahmevorrichtung (6) mit dem optischen Modul auf der Projektionsfläche.

12. Verfahren zum Betreiben eines Benutzerassistenzsystems (2) nach Anspruch 10 oder 11, **dadurch gekennzeichnet, dass** das Identifikationsmerkmal zumindest ein Teil einer äußeren Erscheinung und/oder einer Form des chirurgischen Instruments (5) oder der Aufnahmevorrichtung (6), und/oder das Identifikationsmerkmal zumindest ein Teil eines an oder auf dem chirurgischen Instrument (5) oder der Aufnahmevorrichtung (6) vorhandenen maschinenlesbaren Codes ist.

13. Computerprogrammprodukt, welches eine Datenbrille (14) in einem Benutzerassistenzsystem (2) nach einem der Ansprüche 1 bis 8 dazu veranlasst, ein Verfahren nach einem der Ansprüche 10 bis 12 auszuführen.

## Claims

1. A user assistance system (2) of a cleaning and disinfection device (4) for cleaning and disinfecting surgical instruments (5), wherein the user assistance system (2) comprises data eyeglasses (14) which comprise a camera (16) and an imaging optical module with a projection surface, wherein the imaging optical module is configured to project information on the projection surface arranged in the field of vision of the user of the data eyeglasses (14), and wherein the user assistance system (2) furthermore comprises a data storage (28) in which a plurality of data sets on different types of surgical instruments (5) are stored, wherein information relating to an identifying feature of a particular type of surgical instrument (5) and visual data relating to instructions on cleaning this type are in each data set, and wherein the user assistance system (2) is furthermore configured to capture with the camera (16) an image of a surgical instrument (5) to be cleaned, to compare the image data of the image with the identifying features in the data sets, to identify a type of the surgical instrument (5) to be cleaned using an identifying feature in the image, and to display visual information relating to instructions on cleaning this type on the projection surface by means of the optical module, wherein the visual information are:
an explanatory text
and/or a picture or a sketch that explains the manipulations to perform and/or handing instructions in the sense of augmented reality, in which in particular relevant areas are highlighted in color and/or marked with an illuminated frame and/or a correct position of the surgical instrument (5) is superimposed with the image visible to the user,
**characterized in that** the data-eyeglasses (14) further comprise an audio output apparatus, and audio data comprising instructions on cleaning the surgical instrument (5) that supplement the visual data are in at least one data set, and/or at least one additional data set.

2. The user assistance system (2) according to claim 1, **characterized in that** a plurality of additional data sets for different types of holding apparatuses (6), in particular cleaning baskets of cleaning and disinfecting devices (4), are stored in the data storage (28), wherein information relating to an identifying feature of a particular type of holding apparatus (6) and visual information relating to the cleaning of surgical instruments (5) using this type are in each of these data sets, wherein the user assistance system (2) is also configured to additionally capture an image of a holding apparatus (6) with the camera (16), to compare the image data of the additional image with the identifying features in the additional data sets, to identify a type of the holding apparatus (6) using an identifying feature in the image, and to display visual information relating to the cleaning of the surgical instrument (5) using this type of holding apparatus (6) on the projection surface by means of the optical module.

3. The user assistance system (2) according to claim 1 or 2, **characterized in that** the identifying feature is at least part of an external appearance and/or shape of the surgical instrument (5) or the holding apparatus (6), and/or the identifying feature is at least part of a machine-readable code in or on the surgical instrument (5) or holding apparatus (6).

4. The user assistance system (2) according to claim 2 or 3, **characterized in that** the visual information relates to the insertion of the surgical instrument (5) into the holding apparatus, and/or the connection of the surgical instrument (5) to connections of the holding apparatus (6).

5. The user assistance system (2) according to one of claims 1 to 4, **characterized in that** the projection surface is part of a prism (20), wherein along the line of sight, a user of the data eyeglasses (14) can observe both the field of vision as well as information projected on the projection surface through the part of the prism (20) comprising the projection surface.

6. The user assistance system (2) according to one of claims 1 to 5, **characterized in that** the data eyeglasses (14) comprise the data storage (28).

7. The user assistance system (2) according to one of claims 1 to 5, **characterized in that** the data storage (28) is outside of the data eyeglasses (14), and the data eyeglasses (14) comprise a transmitting/receiving unit for establishing a wired or wireless data link (24) with the data storage (28).

8. The user assistance system (2) according to one of claims 1 to 7, **characterized in that** the surgical instrument (5) is an endoscope.

9. A cleaning and disinfecting device (4) comprising a user assistance system (2) according to one of claims 1 to 8.

10. A method for operating a user assistance system (2) according to one of claims 1 to 8, wherein the method comprises the following steps:
- Capture with the camera (16) of an image of a surgical instrument (5) to be cleaned,
- Comparison of the image data of the image with the identifying features in the data sets,
- Identification of a type of surgical instrument (5) to be cleaned using an identifying feature in the image,
- By means of the optical module, displaying on the projection surface visual information relating to instructions for cleaning,
**characterized in that**
the data eyeglasses (14) furthermore comprise an audio output apparatus, and audio data comprising instructions on cleaning the surgical instrument (5) that supplement the visual data are in at least one data set, and/or at least one additional data set, and audio data supplementing the visual information are output.

11. The method for operating a user assistance system (2) according to claim 10, **characterized in that** a plurality of additional data sets for different types of holding apparatuses (6), in particular cleaning baskets of cleaning and disinfecting devices (4), are saved in the data storage (28), wherein information relating to an identifying feature of a particular type of holding apparatus (6) and visual information relating to the cleaning of surgical instruments using this type are in each of these data sets, wherein the method furthermore comprises the following steps:
- Capture of an additional image of a holding apparatus (6) with the camera (16),
- Comparison of the additional image with the identifying features in the data sets,
- Identification of a type of holding apparatus (6) using an identifying feature in the image, and
- By means of the optical module, displaying on the projection surface visual information relating to the cleaning of the surgical instrument (5) using this type of holding apparatus (6).

12. The method for operating a user assistance system (2) according to claim 10 or 11, **characterized in that** the identifying feature is at least part of an external appearance and/or shape of the surgical instrument (5) or the holding apparatus (6), and/or the identifying feature is at least part of a machine-readable code in or on the surgical instrument (5) or holding apparatus (6).

13. A computer program product that causes data eyeglasses (14) in a user assistance system (2) according to one of claims 1 to 8 to execute a method according to one of claims 10 to 12.

## Revendications

1. Système (2) d'assistance à un utilisateur d'un dispositif (4) de nettoyage et de désinfection pour le nettoyage et la désinfection d'instruments chirurgicaux (5), le système (2) d'assistance à un utilisateur comprenant des lunettes connectées (14) qui comprennent une caméra (16) et un module optique d'imagerie avec une surface de projection, le module optique d'imagerie étant conçu pour projeter des informations sur la surface de projection disposée dans le champ de vision d'un utilisateur des lunettes connectées (14), et le système (2) d'assistance à un utilisateur comprenant en outre une mémoire de données (28) dans laquelle sont stockés une pluralité d'ensembles de données pour différents types d'instruments chirurgicaux (5), chaque ensemble de données contenant des informations relatives à une caractéristique d'identification d'un type particulier d'instrument chirurgical (5) et des données visuelles relatives à des instructions pour le nettoyage de ce type, et le système (2) d'assistance à un utilisateur étant en outre agencé pour capturer une image d'un instrument chirurgical (5) à nettoyer avec la caméra (16), pour comparer les données d'image de l'image avec les caractéristiques d'identification dans les ensemble de données, pour identifier un type d'instrument chirurgical (5) à nettoyer à partir d'une caractéristique d'identification présente dans l'image et pour afficher des informations visuelles relatives à des instructions pour le nettoyage de ce type avec le module optique sur la surface de projection, les informations visuelles étant :
un texte explicatif
et/ou une image ou un schéma expliquant les manipulations à effectuer et/ou une instruction d'action dans le sens d'une réalité augmentée, dans laquelle des zones particulièrement pertinentes sont mises en évidence par des couleurs et/ou marquées par des cadres lumineux et/ou une position correcte de l'instrument chirurgical (5) est superposée à l'image visible par l'utilisateur,
**caractérisé en ce que**
les lunettes connectées (14) comprennent en outre un dispositif de sortie audio, et des données audio comprenant des instructions pour le nettoyage de l'instrument chirurgical (5) sont présentes dans au moins un ensemble de données et/ou au moins un autre ensemble de données complétant les données visuelles.

2. Système (2) d'assistance à un utilisateur selon la revendication 1, **caractérisé en ce que** la mémoire de données (28) contient en outre une pluralité d'autres ensembles de données pour différents types de dispositifs de réception (6), en particulier des paniers de nettoyage de dispositifs (4) de nettoyage et de désinfection, chacun de ces ensembles de données contenant des informations relatives à une caractéristique d'identification d'un type déterminé de dispositif de réception (6) et des informations visuelles relatives au nettoyage d'instruments chirurgicaux (5) en utilisant ce type, le système (2) d'assistance à un utilisateur étant en outre conçu pour acquérir en plus une image d'un dispositif de réception (6) avec la caméra (16), pour comparer les données d'image de l'image supplémentaire avec les caractéristiques d'identification dans les autres ensemble de données, pour identifier un type de dispositif de réception (6) en utilisant une caractéristique d'identification présente dans l'image et pour afficher des informations visuelles relatives au nettoyage de l'instrument chirurgical (5) en utilisant ce type de dispositif de réception (6) avec le module optique sur la surface de projection.

3. Système (2) d'assistance à un utilisateur selon la revendication 1 ou la revendication 2, **caractérisé en ce que** la caractéristique d'identification est au moins une partie d'un aspect extérieur et/ou d'une forme de l'instrument chirurgical (5) ou du dispositif de réception (6), et/ou la caractéristique d'identification est au moins une partie d'un code lisible par machine présent dans ou sur l'instrument chirurgical (5) ou le dispositif de réception (6).

4. Système (2) d'assistance à un utilisateur selon la revendication 2 ou la revendication 3, **caractérisé en ce que** les informations visuelles sont relatives à la mise en place de l'instrument chirurgical (5) dans le dispositif de réception (6) et/ou le raccordement de l'instrument chirurgical (5) à des raccords du dispositif de réception (6).

5. Système (2) d'assistance à un utilisateur selon l'une des revendications 1 à 4, **caractérisé en ce que** la surface de projection est une partie d'un prisme (20), un utilisateur des lunettes connectées (14) étant apte à observer à la fois le champ de vision et les informations projetées sur la surface de projection dans la direction d'observation à travers la partie du prisme (20) comprenant la surface de projection.

6. Système (2) d'assistance à un utilisateur selon l'une des revendications 1 à 5, **caractérisé en ce que** la mémoire de données (28) est comprise dans les lunettes connectées (14).

7. Système (2) d'assistance à un utilisateur selon l'une des revendications 1 à 5, **caractérisé en ce que** la mémoire de données (28) est présente à l'extérieur des lunettes connectées (14) et **en ce que** les lunettes connectées (14) comprennent une unité d'émission/réception pour établir une liaison de données (24) filaire ou sans fil avec la mémoire de données (28).

8. Système (2) d'assistance à un utilisateur selon l'une des revendications 1 à 7, **caractérisé en ce que** l'instrument chirurgical (5) est un endoscope.

9. Dispositif (4) de nettoyage et de désinfection comprenant un système (2) d'assistance à un utilisateur selon l'une des revendications 1 à 8.

10. Procédé de mise en oeuvre d'un système (2) d'assistance à un utilisateur selon l'une quelconque des revendications 1 à 8, ledit procédé comprenant les étapes suivantes :
- acquisition au moyen de la caméra (16) d'une image d'un instrument chirurgical (5) à nettoyer,
- mise en correspondance des données d'image de l'image avec les caractéristiques d'identification dans les ensembles de données,
- identification d'un type d'instrument chirurgical (5) à nettoyer en utilisant une caractéristique d'identification présente dans l'image,
- affichage sur l'écran de projection, au moyen du module optique, des informations visuelles relatives à des instructions de nettoyage de ce type,
**caractérisé en ce que**
les lunettes connectées (14) comprennent en outre un dispositif de sortie audio et, dans au moins un ensemble de données et/ou au moins un autre ensemble de données, sont présentes des données audio qui complètent les données visuelles comprenant des instructions pour le nettoyage de l'instrument chirurgical (5), et les données audio complétant les informations visuelles sont émises.

11. Procédé de mise en oeuvre d'un système (2) d'assistance à un utilisateur selon la revendication 10, **caractérisé en ce qu'**une pluralité d'autres ensembles de données pour différents types de dispositifs de réception (6) est en outre contenue dans la mémoire de données (28), en particulier pour des paniers de nettoyage de dispositifs (4) de nettoyage et de désinfection, des informations relatives à une caractéristique d'identification d'un type particulier de dispositif de réception (6) étant contenues dans chacun de ces ensembles de données, ainsi que des informations visuelles relatives au nettoyage d'instruments chirurgicaux (5) en utilisant ce type, le procédé comprenant en outre les étapes suivantes :
- capture au moyen de la caméra (16) d'une image supplémentaire d'un dispositif de réception (6),
- mise en correspondance des données d'image de l'image supplémentaire avec les caractéristiques d'identification dans les autres ensemble de données,
- identification d'un type de dispositif de réception (6) en utilisant une caractéristique d'identification présente dans l'image et
- affichage sur la surface de projection, au moyen du module optique, d'informations visuelles relatives au nettoyage de l'instrument chirurgical (5) en utilisant ce type de dispositif de réception (6).

12. Procédé de mise en oeuvre d'un système (2) d'assistance à un utilisateur selon la revendication 10 ou la revendication 11, **caractérisé en ce que** la caractéristique d'identification est au moins une partie d'un aspect extérieur et/ou d'une forme de l'instrument chirurgical (5) ou du dispositif de réception (6), et/ou la caractéristique d'identification est au moins une partie d'un code lisible par machine présent dans ou sur l'instrument chirurgical (5) ou le dispositif de réception (6).

13. Produit programme d'ordinateur permettant à des lunettes connectées (14) dans un système (2) d'assistance à un utilisateur selon l'une quelconque des revendications 1 à 8, de mettre en oeuvre un procédé selon l'une quelconque des revendications 10 à 12.
